Europäisches Patentamt

European Patent Office

Office européen des brevets

(11) Veröffentlichungsnummer: **0 545 242 A1**

## EUROPÄISCHE PATENTANMELDUNG

(21) Anmeldenummer: **92120049.9**

(51) Int. Cl.5: **A61B 5/00**, G01N 27/416

(22) Anmeldetag: **25.11.92**

(30) Priorität: **28.11.91 DE 4139122**

(43) Veröffentlichungstag der Anmeldung:
**09.06.93 Patentblatt 93/23**

(84) Benannte Vertragsstaaten:
**AT CH DE DK ES FR GB IT LI NL SE**

(71) Anmelder: **Fenzlein, Paul-Gerhard**
**Elsa-Brandströmstrasse 33**
**W-8500 Nürnberg 80(DE)**

(72) Erfinder: **Fenzlein, Paul-Gerhard**
**Elsa-Brandströmstrasse 33**
**W-8500 Nürnberg 80(DE)**
Erfinder: **Anderer, Wolfgang, Dr.**
**Steinstrasse 8**
**W-8501 Aurachtal(DE)**

(74) Vertreter: **Rau, Manfred, Dr. Dipl.-Ing. et al**
**Patentanwälte Rau, Schneck & Hübner,**
**Königstrasse 2**
**W-8500 Nürnberg 1 (DE)**

(54) Vorrichtung zur Bestimmung medizinischer, organ- oder stoffwechselfunktionsrelevanter, elektrochemischer Messgrössen.

(57) Eine Meßvorrichtung zur Bestimmung medizinischer, Organ- oder Stoftwechsel-funktionsrelevanter, elektrochemischer Meßgrößen umfasst einen Sensorkopf (1), ein Kopplungsteil (2) an dem der Sensorkopf (1) auswechselbar befestigt ist und ein mikroprozessor-gesteuertes Basisgerät (5), das versehen ist mit einer zentralen Steuereinheit (Mikroprozessor 6), mit einer Meßwerterfassungs- und -auswerteeinrichtung (7) zur Erfassung und Umrechnung der vom Kopplungsteil (2) herangeführten Meßsignale entsprechend einer jeweils zugeordneten Kalibriervorschrift, mit einer Anzeigeeinheit (8) zur Anzeige von Meßwerten und mit einer Tastatur (11) zur Eingabe meßrelevanter Informationen. Der Sensorkopf (1) ist mit seinen Meßelektroden herstellungsseitig standardisiert vorkalibriert, wobei die jeweils einer bestimmten Meßelektrode zugeordneten Kalibriervorschriften im Basisgerät (5) abgespeichert sind und beim Meßeinsatz eines Sensorkopfes (1) die Meßwerterfassungs- und -auswerteeinrichtung (7) durch eine entsprechende externe Eingabe am Basisgerät gesteuert auf die der jeweils im Sensorkopf (1) vorhandenen Meßelektrode zugeordnete Kalibriervorschrift zugreift.

FIG. 1

Die Erfindung betrifft eine Vorrichtung zur Bestimmung medizinischer, Organ- oder Stoffwechsel-funktionsrelevanter, elektrochemischer Meßgrößen nach dem Oberbegriff des Patentanspruchs 1.

Grundsätzlich existieren in der Medizin und insbesondere in der Notfall-, Intensiv-Medizin und der Chirugie objektive Meßparameter zur Bestimmung der Organ- und Stoffwechselfunktionen. Bei diesen Parametern handelt es sich beispielsweise um elektrochemische Größen. Als Beispiel wird auf den sehr wichtigen, unter Beteiligung von Sauerstoff in Geweben und Organen stattfindenden Stoffwechsel im Rahmen der Sauerstoffversorgung von Körperzellen hingewiesen. Im Rahmen der Sauerstoffversorgung des Organismus und davon abhängiger Effekte kann es beispielsweise in einem Gewebe zu Sauerstoffmangel kommen, was zu einer Veränderung der Elektrolytaktivitäten an der Zellmembran führt (Ischämie). Mit einem solchen Sauerstoffmangel geht ein Anstieg der Kaliumaktivität außerhalb der Zelle bei gleichzeitiger Erniedrigung der Natriumaktivität und einem Abfall des Gewebe-pH-Wertes einher. Kalium- und Natriumaktivitäten sowie der Gewebe-pH-Wert sind also mögliche elektrochemische Meßgrößen für die Bestimmung der Sauerstoffversorgung eines Gewebes. Darüberhinausgehende Veränderungen können ebenfalls auf dem Wege der Elektro-Myelographie (EMG) mit Hilfe von Enzym-Elektroden zur Erfassung von Glucose, Lactat oder ähnlichen Stoffwechselprodukten durch ampereometrische Sensoren erfaßt werden.

Als weiteres Beispiel sind pathologisch bedingte Wasseransammlungen im Gewebe (Ödeme) zu nennen, wodurch es zu Störungen der Mikrozirkulation, d.h. der Kapillardurchblutung, einer pathologischen Veränderung in der Kapillarwand und dem Zwischenzellraum (Interstitium) und somit zu ähnlichen Stoffwechselveränderungen kommt, wie bereits oben beschrieben. Auch hier können die oben genannten Meßverfahren gute Hinweise für Diagnose und Therapie geben.

Zur Bestimmung der vorstehend beispielhaft erwähnten elektrochemischen Meßgrößen sind geeignete Meßverfahren und -vorrichtungen auf potentiometrischer bzw. ampereometrischer Basis bereits seit langem grundsätzlich bekannt. Allerdings fanden diese Meßverfahren und -vorrichtungen im wesentlichen nur im Rahmen von Laboruntersuchungen Anwendung, wozu Proben des interessierenden Gewebes zur Verfügung stehen müssen.

Demgegenüber ist es insbesondere im Hinblick auf die Notfall- und IntensivMedizin sowie die Chirurgie erstrebenswert, solche Meßverfahren mit Hilfe geeigneter Meßvorrichtungen "in vivo" - also beispielsweise direkt im Verlauf eines medizinischen Eingriffes - anzuwenden, damit der Chirurg wichtige Sofortinformationen über die Effizienz seiner Maßnahmen und den Zustand des behandelten Organs im Rahmen einer "on-line"-Diagnostik erhält.

Eine zu diesem Zweck geschaffene Vorrichtung der gattungsgemäßen Art ist bereits aus DE-PS 37 25 597 bekannt. Bei dieser Meßvorrichtung zur Messung der Aktivität von Ionen kann ein Sensorkopf mit miniaturisierten, jeweils auf die zu bestimmende Ionenart abgestimmten Meßelektroden "in vivo" auf die Oberfläche eines Gewebes, also beispielsweise eine Organoberfläche, aufgesetzt und die Aktivität interessierender Ionen mit Hilfe von ionenselektiven Membranen im Bereich der Meßelektroden gemessen werden.

Bei einem Kontakt der ionenselektiven Membran mit dem die entsprechenden Ionen enthaltenden Medium kann über Ableitungen von der Membran eine elektromotorische Kraft bzw. ein elektrochemisches Potential EMF gegenüber einer Bezugselektrode gemessen werden, das auch durch die nachfolgend qualitativ wiedergegebene NERNSTsche Gleichung

$$EMF = E_0 - C\,T\,\lg a_{ion}$$

mit $E_0$: Referenzpotential, C: Skalierungsfaktor, T: Temperatur und $a_{ion}$: Ionenaktivität gegeben ist.

Aufgrund dieser Beziehung kann die Ionenaktivität aufgrund einer potentiometrischen Messung ermittelt werden.

Die grundsätzlich aus der vorgenannten Druckschrift bekannte Meßvorrichtung bringt in ihrer praktischen Anwendung Probleme insbesondere hinsichtlich der Kalibierung und der nachfolgenden Messungen mit sich. Jede Meßelektrode des Sensors ist nämlich vor dem eigentlichen Meßeinsatz in zeitaufwendiger Weise zu kalibrieren, d.h. es ist eine Eichkurve der elektromotorischen Kraft in Abhängigkeit der Ionenaktivität bei einer oder mehreren bestimmten Temperaturen zu erstellen. Aufgrund dieser Eichkurven kann anschließend eine bei einer entsprechenden Temperatur gemessene elektromotorische Kraft in eine entsprechende Ionenaktivität umgerechnet werden. Die notwendige Kalibrierung stellt eine komplizierte Meßvorbereitung dar, die zeitaufwendig ist und somit einer wirklichen "on-line"-Diagnostik entgegensteht. Darüberhinaus mußten bei dem bekannten Sensor zwischen den Messungen regelmäßig Nachkalibrierungen zur Meßwert-Korrektur erfolgen. Außerdem ist eine Messung in unterschiedlichen Temperaturbereichen ohne vorherige Kalibrierung bei der entsprechenden Meßtemperatur mit der bekannten Meßvorrichtung nicht möglich, was einen Einsatz in der Organ-Transplantationschirurgie, bei der Spenderorgane im Lagerzustand stark gekühlt sind, sehr erschwert.

Ausgehend von den geschilderten Problemen bei Meßvorrichtungen nach dem Stande der Technik liegt der Erfindung die Aufgabe zugrunde, eine Meßvorrichtung der gattungsgemäßen Art so weiterzubilden, daß eine einfache, automatisierte und schnelle Messung unterschiedlichster Meßgrößen ohne nennenswerten Kalibrieraufwand möglich wird.

Diese Aufgabe wird durch die im Kennzeichnungsteil des Patentanspruches 1 angegebenen Merkmale gelöst. Demnach ist der Sensorkopf mit seinen Meßelektroden herstellungsseitig standardisiert vorkalibriert, d.h. die Meßelektroden verfügen über fest definierte elektrochemische Eigenschaften. Durch diese Standardisierung können den jeweiligen Meßelektroden zugeordnete Kalibriervorschriften im Basisgerät in einem entsprechenden Speicher abgelegt sein, wodurch sich beim Meßeinsatz eines Sensorkopfes eine aufwendige Kalibrierung erübrigt. Um bei einem Meßeinsatz eines Sensorkopfes eine korrekte Umrechnung der gemessenen Potentialwerte z.B. in eine Ionenaktivität zu ermöglichen, genügt es, durch eine entsprechende externe Eingabe am Basisgerät gesteuert der Meßwerterfassungs- und -auswerteeinrichtung des Basisgerätes jeweils mitzuteilen, welche Art von Meßelektroden im Sensorkopf integriert sind und auf welche der gespeicherten Kalibriervorschriften somit bei der Umrechnung zuzugreifen ist.

Durch die vorstehend erläuterten Maßnahmen wird der Einsatz einer erfindungsgemäßem Meßvorrichtung und der ihr zugehörigen Sensoren und Meßelektroden in vielen Bereichen der Medizin möglich, da eine solche Meßvorrichtung besonders einfach zu bedienen ist und eine schnelle Messung erlaubt. Diagnosen, Therapien und Operationen werden durch den Einsatz der Meßvorrichtung nicht verlängert oder behindert.

Die Steuerung des Zugriffs der Meßwerterfassungs- und -auswerteeinrichtung auf die dem jeweiligen Sensorkopf entsprechende Kalibriervorschrift kann entweder durch Eingabe eines für jeden Sensor repräsentativen Codes über die Tastatur des Basisgerätes (Anspruch 2) oder durch Einlesen eines auf den Sensor oder dessen Verpackung aufgebrachten Strich- oder Barcodes mittels eines mit dem Basisgerät verbundenen Strich-Code-Lesers (Anspruch 3) erfolgen. Zur Codierung oder Speicherung entsprechender Informationen sind auch Magnetstreifen oder in den Sensorkopf integrierte, von außen abrufbare Speicherchips möglich.

Eine besonders hohe Meßgenauigkeit insbesondere innerhalb eines weiten Temperaturbereiches beispielsweise von knapp oberhalb des Gefrierpunktes bis zu Körpertemperatur wird durch die im Anspruch 4 angegebenen Maßnahmen erzielt. Dabei wird die Erkenntnis ausgenutzt, daß die elektrochemischen Eigenschaften einer jeden Meßelektrode über ihr $E_0$-Potential und den Skalenfaktor C als Parameter definiert sind, in die die herstellungsseitige Auslegung der miniaturisierten Meßelektroden eingeht. Diese beiden Parameter bestimmen den sogenannten Isothermenpunkt. Liegt dieser innerhalb des der Elektrode zugewiesenen Aktivitäts-Meßbereich, so kann bei Messungen in unterschiedlichsten Temperaturbereichen eine Umrechnung des Potentialwertes in einen entsprechenden Aktivitätswert von Ionen mit einem besonders geringen Skalierungsfehler erfolgen. Damit ist für die Temperatur-Korrektur bei Messungen von Ionenaktivitäten oder auch Stoffwechselprodukten (z.B. Bicarbonat, $pCO_2$, $pO_2$ u.a.) keine gesonderte Kalibrierung notwendig, da über die eingangs erwähnte Standardisierung der Meßelektroden alle notwendigen Daten über das Referenzpotential $E_0$ in der NERNSTschen Gleichung und den entsprechenden Isothermen-Punkt jeder betreffenden Meßelektrode dem Meßsystem über die Sensor-Codierung bekannt sind.

Die gemäß Anspruch 5 vorgesehene Nachkalibrierung der erfindungsgemässen Vorrichtung stellt eine Kontrollmaßnahme aus Sicherheitsgründen dar.

Bei der Nachkalibrierung mittels geeichter Referenzlösungen bei einer festen Temperatur erkennt die Meßvorrichtung nämlich automatisch über einen Soll-Ist-Vergleich, ob die Meßelektroden des jeweils verwendeten Sensorkopfes sich tatsächlich entsprechend der standardisierten Vorkalibrierung verhalten. Ergeben sich bei dem Soll-Ist-Vergleich aufgrund von Instabilitäten der Meßelektroden lediglich leichte Abweichungen im Sinne eines additiven Offsets in der Abhängigkeit des gemessenen Potentialwertes von der jeweiligen Aktivität, so kann diese Abweichung - sofern sie sich innerhalb eines Toleranzbereiches bewegt - rechnerisch von dem Meßwerterfassungs- und -auswerteeinrichtung kompensiert werden. Liegen die bei der Nachkalibrierung ermittelten Ist-Werte untragbar weit entfernt von dem Soll-Werten, so kann die Meßvorrichtung selbsttätig die Messung unterbinden oder zumindest eine entsprechende Warnung ausgeben, daß der gerade eingesetzte Sensorkopf nicht funktionsfähig ist.

Vorteilhafterweise können in den Sensorkopf weitere physikalische Sensoren, wie Druckaufnehmer, Strömungsmesser, optische Meßeinrichtungen und Elektro-Myelographie-Elektroden integriert sein. Dadurch wird die erfindungsgemäße Vorrichtung zu einem multifunktionalen Meßsystem, daß unterschiedlichsten Meßaufgaben gewachsen ist, wobei auch hier durch eine herstellungsseitige Vorkalibrierung der Kalibrieraufwand zu Null gemacht oder zumindest soweit reduziert wird, daß er für den schnellen Einsatz der Sensoren nicht hinderlich ist. Hier sind z.B. Strömungsmessungen mit Hilfe miniaturisierter Ultraschall-Sensoren zu nennen. Weist ein erfindungsgemäßer Sensorkopf sowohl miniaturisierte Membran-Elektroden zur potentiometrischen Messung der Elektrolytaktivität als auch einen miniaturisierten Flowmeter in Form eines Ultraschall-Sensors auf, so kann beispielsweise in Transplantationsorganen bei der Entnahme des Organs aus dem Spenderkörper, bei dessen Lagerung und Implantation in den Empfängerkörper die Mikrozirkulation von Blut oder extra- bzw. intrazellulären Perfusionslösungen in bestimmten Gewebebereichen des Organs sowohl über die Strömungsmessung als auch die Elektrolytaktivitätsmessung unabhängig voneinander überwacht werden. Damit ist also eine sichere, da auf unterschiedliche Parameter zugreifende Überwachung eines solchen Organs möglich. In diesem Zusammenhang ist zu ergänzen, daß auch ein entsprechender Strömungsmesser ohne besonderen Aufwand nachkalibriert werden kann, da bei der vorstehend bereits erwähnten Nachkalibrierung mittels geeichter Referenzlösungen innerhalb dieser die Strömung gleich Null ist und somit der bei der Referenzmessung ermittelte Strömungswert ohne weiteres auf den Wert Null abgeglichen werden kann.

Die Ansprüche 8 und 9 kennzeichnen unterschiedliche Alternativen für die Ausbildung des Kopplungsteils der Meßvorrichtung. So kann das Kopplungsteil einerseits über eine mehradrige elektrische Kabelverbindung mit dem Basisgerät verbunden sein. Vorzugsweise ist das Kopplungsteil dann als Handgriff ausgebildet.

Die in Anspruch 9 angegebene Ausgestaltung stellt eine Besonderheit dar. Das Kopplungsteil mit einem Sensorkopf ist dabei als implantierbarer Meßfühler ausgebildet, mit dem eine kontinuierliche Überwachung der wichtigsten Organ- und Stoffwechselfunktionen beispielsweise bei einem Schwerstkranken auf der Intensivstation ohne nennenswerte Belastung für den Patienten und ohne Einschränkung für das medizinische Personal möglich wird. Der Meßfühler stellt dabei eine komplexe Einheit dar, in die eine geeignete Spannungsversorgung und eine Meßelektronik mit Meßverstärker zur Impedanzwandlung, AD-Wandler und Meßwert-Umsetzung sowie eine geeignete Telemetrie-Übertragungseinrichtung integriert ist. Die durch den Sensorkopf gemessenen Meßgrößen werden dabei im Kopplungsteil in eine telemetrisch übertragbare Form konvertiert und anschließend über die telemetrische Sendeeinrichtung im Kopplungsteil zu der telemetrischen Empfangseinrichtung im Basisgerät übertragen.

Durch eine Ausgestaltung der erfindungsgemäßen Vorrichtung nach Anspruch 10 ist es möglich, mit deren Hilfe auch im Blut zu messen, ohne hierbei das Blut nach dem Meßvorgang verwerfen zu müssen.

Weitere Merkmale, Einzelheiten und Vorteile der Erfindung sind der nachfolgenden Beschreibung entnehmbar, in der ein Ausführungsbeispiel anhand der beiliegenden Figuren näher erläutert wird.

Es zeigen:

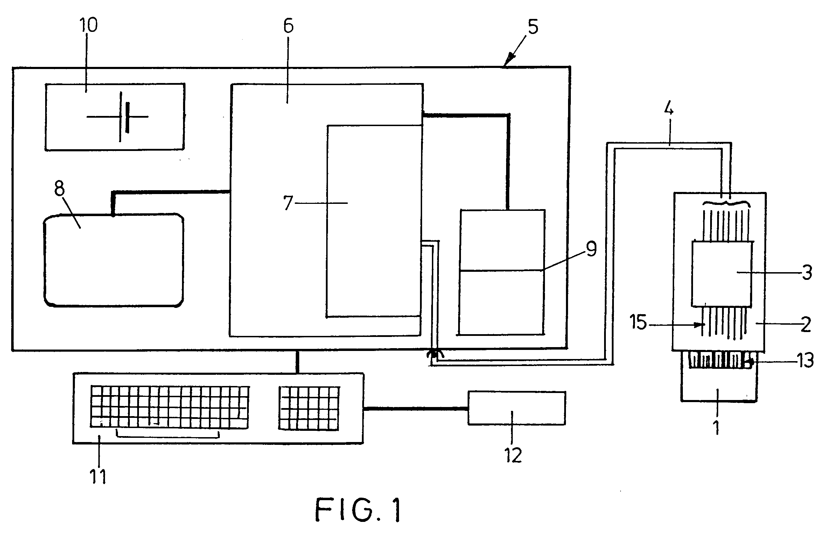

Fig. 1    ein Blockdiagramm einer Meßvorrichtung gemäß der Erfindung,

Fig. 2    einen schematischen Längsschnitt durch einen Sensorkopf der Vorrichtung gemäß Fig. 1

Fig. 3    eine Ansicht der Stirnseite des Sensorkopfes gemäß Fig. 2 und

Fig. 4    ein grafisches Schaubild zur Darstellung der Abhängigkeit eines Meßpotentials von der Ionenaktivität und der Temperatur.

Eine erfindungsgemäße Meßvorrichtung zur Bestimmung medizinischer, Organ- oder Stoffwechsel-funktionsrelevanter, elektrochemischer Meßgrößen weist einen Sensorkopf 1 auf, der an einem als Handgriff ausgebildeten Kopplungsteil 2 auswechselbar über eine Steckverbindung befestigt ist. Im Tastkopf ist ein Meßverstärker 3 zur Verstärkung und Impedanzwandlung der in der Regel hochohmigen Meßsignale vom Sensorkopf 1 integriert. Das Kopplungsteil 2 steht über ein mehradriges Kabel 4 mit dem Meßeingang eines Basisgerätes 5 in Verbindung, das als zentrale Steuereinheit einen Mikroprozessor 6 mit entsprechender Arbeits-, Daten- und Programmspeichern aufweist. Im Basisgerät 5 ist weiterhin eine Meßwerterfassungs- und -auswerteeinrichtung 7 zur Erfassung und Umrechnung der vom Kopplungsteil 2 herangeführten Meßsignale vorgesehen. Weiterhin ist eine Anzeigeeinheit 8 in Form eines Flüssigkristall-Bildschirmes sowie ein Festplatten- und Floppy-Disk-Laufwerk 9 sowie eine übliche Netzspannungsversorgung 10 vorgesehen.

Über eine externe Tastatur 11 können meß- oder patientenrelevante Informationen eingegeben werden. Über diese Tastatur 11 ist weiterhin mit dem Basisgerät 5 ein Strich-Code-Leser 12 verbunden, mittels dem die in dem Strich-Code 13 auf dem Sensorkopf 1 niedergelegten Informationen über die Meßelektroden des Sensorkopfes 1 in das Basisgerät 5 eingespeist werden können.

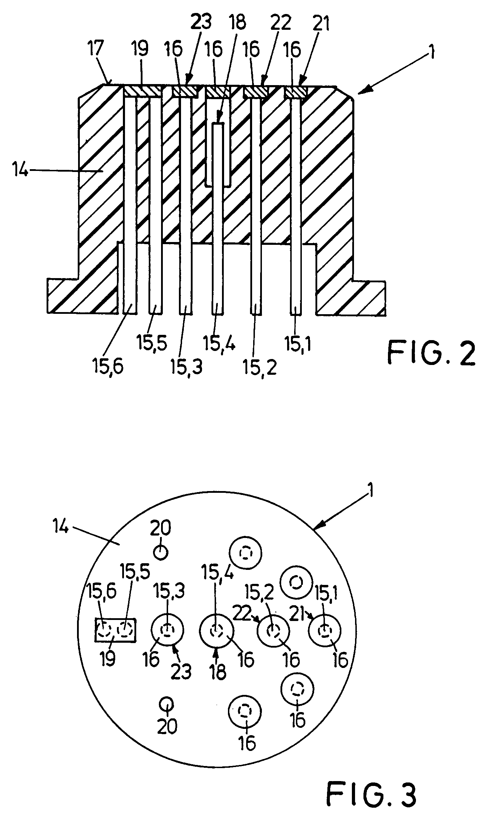

Der in Fig. 2 und 3 gezeigte Sensorkopf besteht aus einem im wesentlichen zylindrischen Körper 14 aus PVC- oder Polyurethan-Vollkunststoffmaterial, in das verschiedene elektrische Ableitungen 15 in Form von Silberdrähten eingebettet sind. Auch andere Edelmetalle, wie Gold oder Platin, können für die Ableitungen 15 verwendet werden. Die Ableitungen 15.1, 15.2 und 15.3 bilden mit ionenselektiven Membranen 16, die in Vertiefungen in der Stirnseite 17 des Körpers 14 eingefügt sind, ionenselektive Meßelektroden für potentiometrische oder ampereometrische Messungen. Bei den Membranen 16 handelt es sich um PVC-Matrix-Membranen. Zentral in den Körper 14 ist eine Bezugselektrode 18 angeordnet, deren Bezugspotential über die Ableitung 15.4 dem Kopplungsteil 2 und damit der Meßwerterfassungs- und -auswerteeinrichtung 7 im Basisgerät 5 zuführbar ist. Über die Ableitungen 15.5 und 15.6 ist als Temperatursensor ein Pt-100-Widerstand 19 angeschlossen. Wie aus Fig. 3 deutlich wird, sind weitere Sensoren 20 beispielsweise für die Elektromyelographie (EMG) oder Enzym-Elektroden zur Bestimmung der Glukose- oder Lactatkonzentration in den Körper 14 eingebettet und enden in der Stirnseite 17.

Die von den Membranen 16 in Verbindung mit den zugehörigen Ableitungen 15.1 bis 15.3 gebildeten Meßelektroden 21,22,23 dienen beispielsweise zur Messung der Aktivität von Kalium- oder Natrium-Ionen und zur Messung des pH-Wertes.

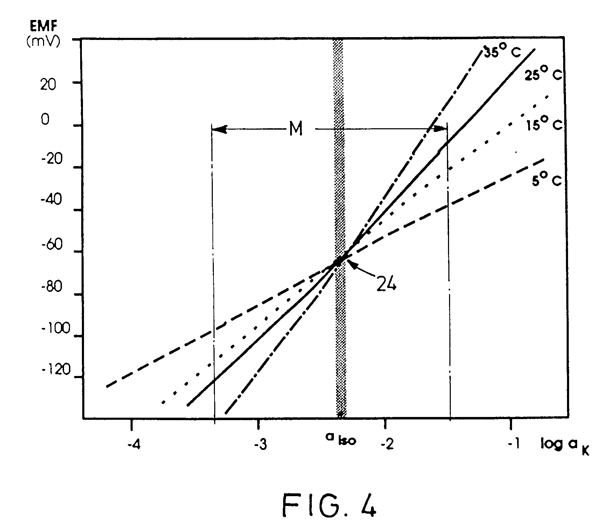

Am Beispiel der Kalium-sensitiven Meßelektrode 21 wird die Funktionsweise der erfindungsgemäßen Vorrichtung nachfolgend erläutert. Die Meßelektrode 21 ist herstellungsseitig so ausgelegt, daß sie die in Fig. 4 dargestellte Abhängigkeit des von ihr gelieferten Meßpotentials EMF von der Ionenaktivität $a_K$ und der Temperatur zeigt. Die herstellungsseitige Auslegung von Meßelektroden im allgemeinen und der Meßelektrode 21 im speziellen mit charakteristischem $E_0$-Potential, Skalierungsfaktor C und Isothermenpunkt 24 wird dabei dadurch gewährleistet, daß einerseits die Ableitungen 15 in ihrer Oberflächengüte und Dimensionierung und andererseits die Membranen 16 in ihrer Dimensionierung und Hochohmigkeit reproduzierbar innerhalb enger Toleranzen liegen. Herstellungsseitig werden also Sensorköpfe 1 mit standardisierten Meßelektroden hergestellt, die eine in Fig. 4 dargestellte Abhängigkeit des gelieferten Meßpotentials EMF von der Ionenaktivität $a_K$ und der Temperatur mit einem charakteristischen $E_0$-Potential, Skalierungsfaktor C und Isothermenpunkt 24 zeigen. Letzterer ist durch den Schnittpunkt der verschiedenen temperaturabhängigen Meßlinien in dem in Fig. 4 gezeigten Diagramm definiert und liegt etwa in der Mitte des einer Meßelektrode zugewiesenen Aktivitäts-Meßbereiches, der etwa durch den Korridor M gemäß Fig. 4 umrissen ist. Die bei der Vorkalibrierung ermittelten Werte für das $E_0$-Potential und den Skalierungsfaktor C - beide Parameter bestimmen den Isothermenschnittpunkt 24 - werden in codierter Form im Strich-Code 13 niedergelegt und stehen somit als Eingabe-Information für das Basisgerät 5 zur Verfügung.

Als in der Meßwerterfassungs- und -auswerteeinrichtung 7 abgespeicherte Kalibriervorschrift kann dann z.B. die eingangs angegebene NERNSTsche Gleichung in qualitativer Form abgespeichert sein, wobei die die genaue Potential-Aktivitäts-Abhängigkeit definierende Quantifizierung durch ein Ablesen des Strich-Codes 13 vor dem eigentlichen Meßeinsatz des Sensorkopfes 1 vorgenommen wird. Da der Sensorkopf 1 gleichzeitig die bei der Messung herrschende Temperatur messen kann, kann von der Meßwerterfassungs- und -auswerteeinrichtung 7 ein Meßpotential EMF mit Hilfe der gespeicherten Kalibriervorschrift etwa in Form der NERNSTschen Gleichung und der eingelesenen Information über das $E_0$-Potential und den Skalierungsfaktor C die entsprechende Ionenaktivität berechnet und in der Anzeigeeinheit 8 visuell ausgegeben oder zur weiteren Verarbeitung oder Dokumentation auf dem Festplatten- oder Floppy-Disk-Laufwerk 9 abgespeichert werden.

Zusammenfassend kann also mit Hilfe der Vorkalibrierung der Meßelektroden 21 bis 23 eine Messung der Ionenaktivität beliebig innerhalb eines Temperaturbereiches von etwa 0°C bis 40°C erfolgen.

Da die Meßelektroden 21 bis 23 in ihrer Langzeitstabilität einer gewissen Drift unterworfen sind, die sich in einem additiven Offset zu den Meßlinien gemäß Fig. 4 äußert, kann die erfindungsgemäße Meßvorrichtung eine Nachkalibrierung vor dem eigentlichen Meßeinsatz der Meßelektroden 21 bis 23 vornehmen. Bei einer beliebigen Temperatur werden mit Hilfe der jeweiligen Meßelektrode 21 bis 23 in Referenzmessungen bekannter Referenzlösungen Potentialwerte ermittelt, die mit Hilfe der abgespeicherten Kalibriervorschrift in Form z.B. der qualitativ eingespeicherten NERNSTschen Gleichung und der vom Strich-Code eingelesenen Informationen über das $E_0$-Potential und den Skalierungsfaktor C in Ist-Aktivitätswerte umgerechnet und mit den bekannten Soll-Aktivitätswerten des entsprechenden Ions in den Referenzlösungen verglichen werden. Ergibt sich hier lediglich ein additiver Offset innerhalb gewisser enger Toleranzen, so kann die Messung mit der betreffenden Meßelektrode freigegeben und die ermittelten Meßwerte automatisch entsprechend dem ermittelten Offset korrigiert werden. Liegt der Offset außerhalb vorgegebener Grenzen, so kann hier die Messung unterbunden und beispielsweise über die Anzeige eine entsprechende Warnung ausgegeben werden.

**Patentansprüche**

1. Vorrichtung zur Bestimmung medizinischer, Organ- oder Stoffwechselfunktionsrelevanter, elektrochemischer und/oder physikalischer Meßgrößen umfassend:
    - einen Sensorkopf (1) mit miniaturisierten, jeweils auf die zu bestimmenden Meßgrößen abgestimmten Meßelektroden (21,22,23), einem Temperatursensor (Pt-100-Widerstand 19) und einer oder mehreren Bezugselektroden (18),

- ein Kopplungsteil (2), an der der Sensorkopf (1) auswechselbar befestigt ist und das entsprechende Ableitungen (15) zum Wegführen der von den Meßelektroden (21,22,23) und Sensoren (20, Pt-100-Widerstand 19) gelieferten elektrischen Meßsignale aufweist,

- ein Mikroprozessor-gesteuertes Basisgerät (5), das versehen ist mit

-- einer zentralen Steuereinheit (Mikroprozessor 6) mit Arbeits-, Daten- und Programm-Speicher zur Steuerung der geräteinternen Arbeitsabläufe,

-- einer Meßwerterfassungs- und -auswerteeinrichtung (7) zur Erfassung und Umrechnung der vom Kopplungsteil (2) herangeführten Meßsignale entsprechend einer jeweils zugeordneten Kalibriervorschrift,

-- einer Anzeigeeinheit (8) zur Anzeige von der Meßwerterfassungs- und -auswerteeinrichtung (7) ermittelten Meßwerte und

-- einer Tastatur (11) zur Eingabe meßrelevanter Informationen,

**dadurch gekennzeichnet, daß** der Sensorkopf (1) mit seinen Meßelektroden (21,22,23) herstellungsseitig standardisiert vorkalibriert ist und die jeweils einer bestimmten Meßelektrode (21,22,23,) zugeordneten Kalibriervorschriften im Basisgerät (5) abgespeichert sind, wobei beim Meßeinsatz eines Sensorkopfes (1) die Meßwerterfassungs- und -auswerteeinrichtung (7) durch eine entsprechende externe Eingabe am Basisgerät (5) gesteuert auf die den jeweils im Sensorkopf (1) vorhandenen Meßelektroden (21,22, 23) zugeordneten Kalibriervorschriften zugreift.

2. Vorrichtung nach Anspruch 1, **dadurch gekennzeichnet, daß** die Steuerung des Zugriffs der Meßwerterfassungs- und -auswerteeinrichtung (7) auf die entsprechenden Kalibriervorschriften durch Eingabe eines für jeden Sensorkopf (1) bzw. dessen Meßelektroden (21,22,23) repräsentativen Codes über die Tastatur (11) des Basisgerätes (5) erfolgt.

3. Vorrichtung nach Anspruch 1, **dadurch gekennzeichnet, daß** die Steuerung des Zugriffs der Meßwerterfassungs- und -auswerteeinrichtung (7) auf die entsprechenden Kalibriervorschriften durch Einlesen eines auf den Sensorkopf (1) oder dessen Verpak-

kung aufgebrachten oder dort gespeicherten Codes, insbesondere eines Strich-Codes (13), mittels einer mit dem Basisgerät (5) verbundenen Leseeinrichtung, insbesondere einer Strichcode-Leseeinrichtung (12), erfolgt.

4. Vorrichtung nach einem der Ansprüche 1 bis 3, **dadurch gekennzeichnet, daß** der Sensorkopf (1) mit seinen elektrochemischen Meßelektroden (21,22,23) herstellungsseitig derart standardisiert vorkalibriert ist, daß der Isothermenpunkt (24) jeder Meßelektrode (21,22,23) im Aktivitäts-Potential-Referenzdiagramm jeweils innerhalb des der Meßelektrode (21,22,23) zugewiesenen Aktivitätsmeßbereiches M liegt.

5. Vorrichtung nach Anspruch 4, **gekennzeichnet durch** eine vorrichtungsinterne Nachkalibrierung des herstellungsseitig vorkalibrierten Sensorkopfes (1) vor dessen eigentlichen Meßeinsatz, wobei bei einer beliebigen Temperatur in Referenzlösungen gemessene und mit der entsprechenden abgespeicherten Kalibriervorschrift ermittelte Ist-Werte der Aktivität mit den entsprechenden Sollwerten vergleichbar sind.

6. Vorrichtung nach Anspruch 5, **dadurch gekennzeichnet, daß** aus dem Vergleich der Soll- zu den Ist-Werten ein additiver Korrekturwert (Offset) für die Meßwertauswertung durch die Meßwerterfassungs- und -auswerteeinrichtung (7) bei der eigentlichen Messung ableitbar ist.

7. Vorrichtung nach einem Ansprüche 1 bis 6, **dadurch gekennzeichnet, daß** in den Sensorkopf (1) weitere physikalische Sensoren (20), wie Druckaufnehmer, Strömungsmesser, optische Meßeinrichtungen, Elektro-Myelographie-Elektroden und dergleichen integriert sind.

8. Vorrichtung nach Anspruch 1, **dadurch gekennzeichnet, daß** das Kopplungsteil über eine mehradrige elektrische Kabelverbindung (Kabel 4) mit dem Basisgerät (5) verbunden ist.

9. Vorrichtung nach Anspruch 1, **dadurch gekennzeichnet, daß** das Kopplungsteil (2) mit einem Sensorkopf (1) implantierbar ist und eine telemetrische Sendeeinrichtung zur Meßdaten-Übertragung aufweist, die mit einer telemetrischen Empfangseinrichtung im Basisgerät (5) telemetrisch verbunden ist.

**10.** Vorrichtung nach einem der Ansprüche 1 bis 9, **dadurch gekennzeichnet, daß** der Sensorkopf (1) als Durchflußmeßzelle ausgebildet ist.

FIG.1

FIG. 2

FIG. 3

FIG. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 690 147 (A. OOE ET AL) <br> * das ganze Dokument * <br> --- | 1-3,8 | A61B5/00 <br> G01N27/416 |
| Y | EP-A-0 074 498 (F. HOFFMAN-LA ROCHE & CO.) <br> * das ganze Dokument * <br> --- | 1-3,8 | |
| P,X | WO-A-9 217 775 (KODAK LTD.) <br> * das ganze Dokument * <br> --- | 1,2,8 | |
| A | DE-A-3 917 876 (AESCULAP AG) <br> * das ganze Dokument * <br> --- | 1,3 | |
| A | DE-A-3 941 169 (AVL MEDICAL INSTRUMENTS AG) <br> * das ganze Dokument * <br> --- | 1,10 | |
| A | US-A-4 543 955 (E.A.SCHROEPPEL) <br> * das ganze Dokument * <br> ----- | 1,9 | |
| | | | **RECHERCHIERTE SACHGEBIETE (Int. Cl.5)** <br><br> A61B <br> G01N |

Der vorliegende Recherchenbericht wurde für alle Patentansprüche erstellt

| Recherchenort | Abschlußdatum der Recherche | Prüfer |
|---|---|---|
| DEN HAAG | 26 MAERZ 1993 | HUNT B.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

.....................................................................

& : Mitglied der gleichen Patentfamilie, übereinstimmendes Dokument